# EUROPÄISCHE PATENTSCHRIFT

(11) **EP 2 124 850 B1**
(45) Veröffentlichungstag und Bekanntmachung des Hinweises auf die Patenterteilung: **22.12.2010**
(21) Anmeldenummer: 08706879.7
(22) Anmeldetag: 11.02.2008
(51) Int. Cl.: A61F 5/01

(54) **ORTHESE**
ORTHODIC
ORTHÈSE

(30) Priorität: 26.02.2007 DE 102007009605
(43) Veröffentlichungstag der Anmeldung: 02.12.2009
(73) Patentinhaber: Wagner, Helmut, 37115 Duderstadt (DE)
(72) Erfinder: SCHIMEK, Norbert, 37339 Kirchworbis (DE); WAGNER, Helmut, 37115 Duderstadt (DE)
(74) Vertreter: Rehberg Hüppe + Partner
(86) Internationale Anmeldenummer: PCT/DE2008/000258
(87) Internationale Veröffentlichungsnummer: WO 2008/104149

(56) Entgegenhaltungen:
- EP-A- 0 454 186
- WO-A-97/29717
- DE-A1- 4 418 855
- DE-A1- 19 811 925

## Beschreibung

Die Erfindung betrifft eine Orthese, insbesondere eine Knieorthese, mit zwei Lastgelenken, die jeweils einen proximalen und einen distalen Schenkel aufweisen. In anderen Worten betrifft die Erfindung ein Orthesenprinzip für verschiedene Körperteile.

Derartige Orthesen werden zum Schutz entsprechender Bänder vor Überbeanspruchung durch innere oder äußere Krafteinwirkungen so angewendet, dass Bewegungen von Körperteilen eingeschränkt und/oder uneingeschränkt möglich sind. Knieorthesen werden zum Schützen eines Kniegelenks gegen dorsalventrale Knickbewegungen verwendet. Dazu wird die Knieorthese so an einem Oberschenkel und einem Unterschenkel befestigt, dass die beiden Lastgelenke beidseits seitlich des zu schützenden Kniegelenks angeordnet sind. Auftretende Knicklasten werden von der Knieorthese aufgenommen und an dem Knie vorbei in den Ober- bzw. Unterschenkel eingeleitet. Um eine sichere Ableitung von Knickkräften zu gewährleisten, sollten die Lastgelenke dicht am Kniegelenk anliegen.

Knieorthesen werden in der Regel nach Operationen am Knie verwendet, um die noch empfindlichen Bänder des operierten Knies zu schützen. Nach Knieoperationen ist das Gewebe im Bereich des Knies regelmäßig geschwollen und schwillt während des Heilungsprozesses ab. Damit ändert sich die Breite des Knies und der Abstand zwischen den Lastgelenken muss nachgestellt werden, um stets einen optimalen Schutz gegen Knickbewegungen sicherzustellen.

Zur Feineinstellung des Abstandes zwischen den Lastgelenken sind die Knieorthesen zu einem kleinen Teil adaptierbar und werden zusammen mit Distanzelementen verwendet, die zwischen die Lastgelenke und das Knie positioniert werden.

Aus der US 4 886 054 und der DE 44 18 855 A1, der der nächste Stand der Technik darstellt, sind Knieorthesen bekannt, bei der die distalen Schenkel und die proximalen Schenkel jeweils querverschieblich gegeneinander mittels einer Verspanneinrichtung festlegbar sind. Nachteilig hieran ist die aufwändige Konstruktion der Verspanneinrichtung, die zudem wenig benutzerfreundlich ist.

Aus der DE 37 38 664 A1 ist eine Knieorthese bekannt, bei der die proximalen Schenkel mittels Noppen an einer zentralen Lasche befestigt sind. Zum Verändern des Abstands zwischen den Lastgelenken, werden die distalen Schenkel an anderen Noppen befestigt. Nachteilig hieran ist, dass der Abstand nicht stufenlos variierbar ist. Ein weiterer Nachteil ist die geringe Bedienerfreundlichkeit.

Aus der WO 2006/078428 A2 ist eine Knieorthese mit einem Festkörpergelenk bekannt. Nachteilig hieran ist, dass der Abstand zwischen den beiden proximalen Schenkeln nicht festlegbar ist. Aus der EP 1 676 549 A1 ist ein Lastgelenk bekannt. Aus der DE 10 2005 008 340 A1 ist eine Kugelgelenk-Orthese bekannt, bei der die proximalen Schenkel an einer Hülse fest befestigt sind. Aus der DE 297 05 958 U1 ist eine Kniegelenkentlastungsstütze bekannt, bei der die proximalen Schenkel an einer schnürbaren Oberschenkelhülse befestigt sind.

Der Erfindung liegt die Aufgabe zugrunde, eine Orthese, insbesondere eine Knieorthese, vorzuschlagen, die leichter an eine sich ändernde Kniebreite anpassbar ist.

Die Erfindung löst das Problem durch eine Orthese mit den Merkmalen von Anspruch 1.

Vorteilhaft an der Erfindung ist, dass eine stufenlose Anpassung des Abstands zwischen den Lastgelenken an die Kniebreite möglich ist. Schwankt beispielsweise die Schwellung im Knie während des Tagesablaufs, so kann die Orthese leicht nachgestellt werden.

Vorteilhafterweise ist eine erfindungsgemäße Orthese zudem schnell und einfach anpassbar, was zu einer Zeitersparnis sowohl beim Patienten als auch bei medizinischem Fachpersonal führt, das mit der Einstellung der Orthese befasst ist.

Eine erfindungsgemäße Orthese weist zudem eine große Veränderbarkeit des Abstands zwischen den Lastgelenken auf, so dass eine geringere Anzahl an Typen ausreichend ist, um sämtliche auftretende Kniebreiten zu erfassen. Bei dem Vertrieb erfindungsgemäßer Orthesen müssen daher weniger Varianten vorgehalten werden, was Lagerkapazität spart.

Wichtige Komponenten der Orthese, wie beispielsweise die Schenkel, können zudem als Spritzgussteile ausgebildet werden, was zu einer schnellen und kostengünstigen Fertigung auch in großen Stückzahlen führt.

Im Rahmen der vorliegenden Erfindung werden unter den Lastgelenken diejenigen Gelenke der Orthese verstanden, die sich bewegen, wenn das Knie gebeugt oder gestreckt wird.

Bevorzugt ist das Proximal-Gelenk und/oder das Distal-Gelenk ein Drehgelenk, insbesondere ein festlegbares Drehgelenk. Drehgelenke sind besonders einfach und sehr stabil herstellbar und bieten eine hinreichende Festigkeit für die Orthese. Durch ein festlegbares Drehgelenk kann der Abstand zwischen den beiden Lastgelenken stufenlos eingestellt und unmittelbar nach dem Einstellen festgelegt werden. Dadurch wird die Orthese besonders schnell und einfach an eine sich verändernde Kniebreite anpassbar.

In einer bevorzugten Ausführungsform verläuft die Proximal-Gelenk-Schwenkachse durch das Proximal-Gelenk.

Besonders bevorzugt ist das Proximal-Gelenk bzw. das Distal-Gelenk ein Scheren-Drehgelenk. Unter einem Scheren-Drehgelenk ist insbesondere zu verstehen, dass sich die beiden proximalen bzw. distalen Schenkel über das Drehgelenk hinaus erstrecken. Verringert sich der Abstand zwischen den beiden Lastgelenken, so verringert sich bei einem Scheren-Drehgelenk auch der Abstand der beiden Abschnitte der Schenkel, die über das Scheren-Drehgelenk hinausragen.

Alternativ kann das Proximal-Gelenk und/oder das Distal-Gelenk seitlich an den jeweiligen Schenkeln angeordnet sein, so dass sich der Abstand zwischen den über das Drehgelenk hinaus erstreckenden Abschnitten abnimmt, wenn der Abstand zwischen den Lastgelenken zunimmt. Selbstverständlich ist es auch möglich, dass nur das Proximal-Gelenk oder nur das Distal-Gelenk als Scheren-Drehgelenk ausgebildet ist, das andere hingegen nicht.

Bevorzugt ist das Drehgelenk um eine Drehgelenk-Schwenkachse schwenkbar, die senkrecht zu einer Lastgelenk-Schwenkachse verläuft, um die die beiden Lastgelenke schwenkbar sind. Die Drehgelenk-Schwenkachse verläuft dann in dorsal-ventraler Richtung.

In einer bevorzugten Ausführungsform weisen die beiden distalen Schenkel jeweils einen ersten distalen Teilschenkel und einen zweiten distalen Teilschenkel auf und umfassen jeweils ein distales Ausgleichsgelenk, wobei die distalen Ausgleichsgelenke die jeweiligen Teilschenkel um eine Schwenkachse schwenkbar miteinander verbinden, die parallel zu der Drehgelenk-Schwenkachse verläuft. Besonders bevorzugt sind entsprechende Ausgleichsgelenke auch in den proximalen Schenkeln vorgesehen. Diese Ausgleichsgelenke können beispielsweise Scharniergelenke sein. Alternativ sind die Ausgleichsgelenke durch Materialverjüngungen der jeweiligen Schenkel gebildet. Alternativ oder additiv werden die Ausgleichsgelenke durch Bereiche gebildet, in denen das Material einen gegenüber dem umgebenden Material kleineren Elastizitätsmodul aufweist. Wenn die proximalen Schenkel im Proximal-Gelenk gegeneinander verschwenkt werden, so verändert sich der Abstand zwischen den Lastgelenken. Die Ausgleichsgelenke sorgen dafür, dass die beiden Lastgelenke weiter um eine für beide gleiche Schwenkachse schwenken. Dadurch werden die Lastgelenke geschont.

In einer bevorzugten Ausführungsform laufen die proximalen Schenkel vom jeweiligen Lastgelenk aus gesehen hinter dem Proximal-Gelenk in eine Viertelschale aus, so dass beide Viertelschalen eine proximale Halbschale zum Befestigen der Orthese an einem Körperglied bilden. Wenn es sich bei der Orthese um eine Knieorthese handelt, so ist die Halbschale zur Befestigung der Knieorthese an einem Oberschenkel ausgebildet.

Auf analoge Weise laufen die distalen Schenkel vom jeweiligen Lastgelenk aus gesehen bevorzugt hinter dem Distal-Gelenk in eine Viertelschale aus, so dass beide Viertelschalen eine distale Halbschale zur Befestigung der Orthese an einem Körperglied bilden. Wenn es sich bei der Orthese wiederum um eine Knieorthese handelt, dient die distale Halbschale zur Befestigung der Knieorthese an einem Unterschenkel.

Um eine Anpassung an das jeweilige Körperglied zu ermöglichen, sind die proximalen und/oder die distalen Viertelschalen flexibel.

Erfindungsgemäß ist auch eine Ellbogenorthese mit zwei Lastgelenken, die jeweils einen proximalen und einen distalen Schenkel aufweisen. Derartige Ellbogenorthesen werden zum Schützen eines Ellbogengelenks gegen laterale und mediale Knickbewegungen verwendet. Dazu wird die Ellbogenorthese so an einem Oberarm und einem Unterarm befestigt, dass die beiden Lastgelenke beidseits des zu schützenden Ellbogengelenks angeordnet sind. Auftretende Knicklasten werden von der Ellbogenorthese aufgenommen und an dem Ellbogen vorbei in den Ober- bzw. Unterarm eingeleitet. Um eine sichere Ableitung der Knickkräfte zu gewährleisten, sollten die Lastgelenke dicht am Ellbogengelenk anliegen. Ellbogenorthesen werden in der Regel nach Operationen am Ellbogen verwendet, um die noch empfindlichen Bänder des operierten Ellbogens zu schützen. Nach Ellbogenoperationen ist das Gewebe im Bereich des Ellbogens mitunter geschwollen und schwillt während des Heilungsprozesses ab. Damit ändert sich die Breite des Ellbogens und der Abstand zwischen den Lastgelenken muss nachgestellt werden, um stets einen optimalen Schutz gegen Knickbewegungen sicherzustellen.

Werden die Lastgelenke als Kugelgelenke ausgelegt, so können vorteilhafterweise Ausgleichsgelenke entfallen. In diesem Fall sind an den Lastgelenken beispielsweise Pelotten befestigt, so dass die Lastgelenke nicht in unmittelbarem Kontakt mit dem Kniegelenk des Patienten stehen.

Im Folgenden werden Ausführungsformen der Erfindung anhand der Figuren näher erläutert. Dabei zeigt
- Figur 1: eine perspektivische Ansicht einer erfindungsgemäßen Knieorthe- se, bei der sowohl das Proximal-Gelenk als auch das Distal- Gelenk als Scheren-Drehgelenke ausgebildet sind,
- Figur 2: eine alternative Ausführungsform einer erfindungsgemäßen Knieorthese, bei der das Proximal-Gelenk als Drehgelenk, nicht aber als Scheren-Drehgelenk ausgebildet ist,
- Figur 3: eine alternative Ausführungsform einer erfindungsgemäßen Knieorthese, bei der sowohl das Proximal-Gelenk als auch das Distal-Gelenk als Scheren-Drehgelenke ausgebildet sind,

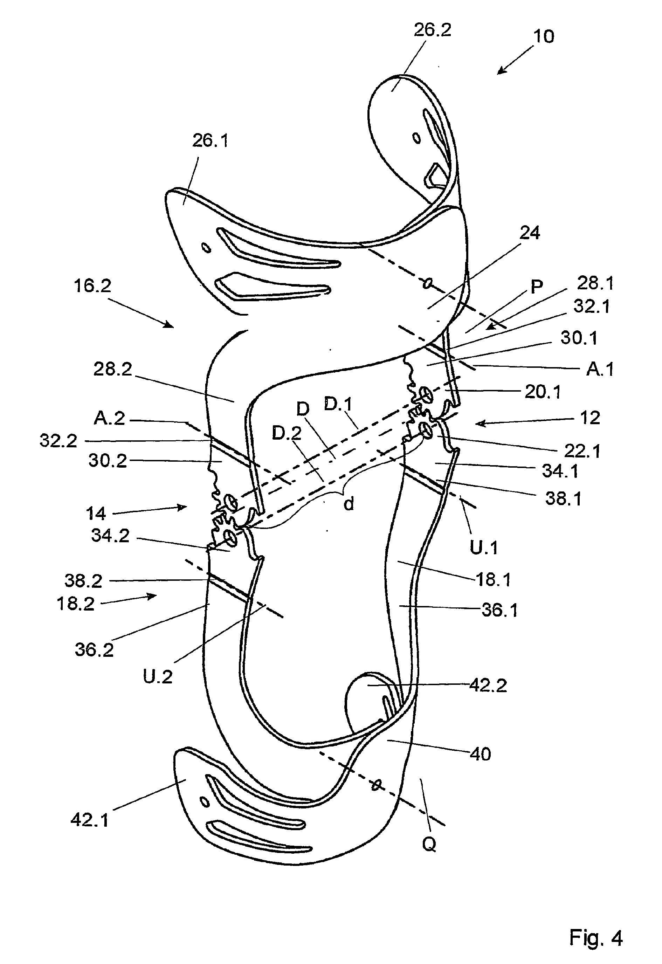

- Figur 4: eine alternative Ausführungsform einer erfindungsgemäßen Knieorthese, bei der das Proximal-Gelenk als Drehgelenk, nicht aber als Scheren-Drehgelenk ausgebildet ist und
- Figur 5: eine weitere alternative Ausführungsform einer erfindungsgemä- β en Knieorthese, bei der das Lastgelenk als Kugelgelenk ausge- bildet ist.

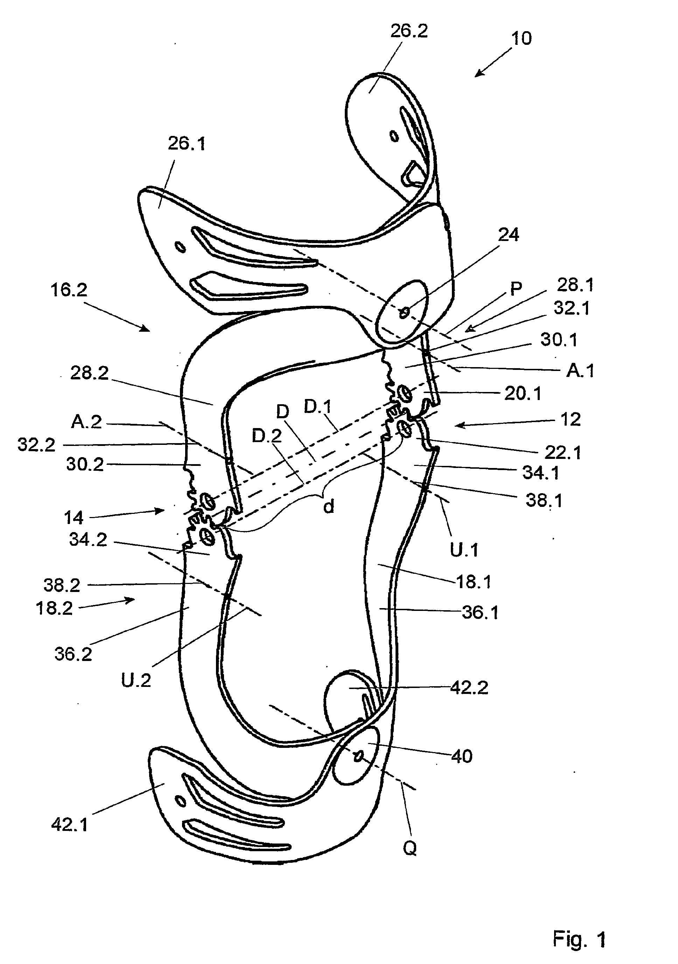

Figur 1 zeigt eine Knieorthese 10 mit einem linken Lastgelenk 12 und einem rechten Lastgelenk 14. Das linke Lastgelenk 12 verbindet einen linken proximalen Schenkel 16.1 mit einem linken distalen Schenkel 18.1 in Form und ist als polyzentrisches Drehgelenk ausgebildet. Das linke Lastgelenk 12 umfasst einen proximalen Gelenkkopf 20.1 und einen distalen Gelenkkopf 22.1, die jeweils Zähne aufweisen. Die Zähne kämmen miteinander, wenn der proximale Schenkel 16.1 relativ zu dem distalen Schenkel 18.1 verschwenkt. Der proximale und der distale Schenkel 16.1 bzw. 18.1 verschwenken in einer zeitlich veränderlichen Lastgelenk-Drehachse D zueinander, die stets parallel zu Verbindungsachsen D1 und D2 verläuft, die die Mittelpunkte der beiden proximalen Gelenkköpfe 20.1., 20.2 bzw. der beiden distalen Gelenkköpfe 22.1, 22.2 miteinander verbinden.

Der proximale Schenkel 16.1 und distale Schenkel 18.1 weisen eine flache, bandartige Gestalt auf und sind durch Spritzgießen hergestellt. Die Knieorthese 10 umfasst zudem einen rechten proximalen Schenkel 16.2 und einen rechten Distalschenkel 18.2, die im Wesentlichen spiegelbildlich zu den linken proximalen Schenkeln 16.1 und dem linken distalen Schenkel 18.1 aufgebaut ist. Um Wiederholungen zu vermeiden, werden im Folgenden nur noch die Komponenten einer Seite beschrieben. Zu jeder derartigen Komponente mit dem Suffix ".1" korrespondiert auf der jeweils anderen Seite eine Komponente, die das Suffix ".2" trägt. ,

Der linke proximale Schenkel 16.1 und der rechte proximale Schenkel 16.2. sind in einem Proximal-Gelenk 24 schwenkbar und gegeneinander festlegbar verbunden und können um eine Proximal-Gelenk-Drehachse P gegeneinander verschwenkt werden. Die Proximal-Gelenk-Drehachse P verläuft senkrecht zu der Lastgelenk-Drehachse D.

Vom linken Lastgelenk 14 aus gesehen erstreckt sich der proximale linke Schenkel 16.1 über das Proximal-Gelenk 24 hinaus und läuft in eine Viertelschale 26.1 aus. Auf gleiche Weise läuft der rechte proximale Schenkel 16.2 in eine Viertelschale 26.2 aus. Wenn die beiden proximalen Schenkel 16.1, 16.2 gegeneinander so verschwenkt werden, dass der Abstand d zwischen dem linken Lastgelenk 12 und dem rechten Lastgelenk 14 abnimmt, so nimmt auch der Abstand zwischen den Viertelschalen 26.1 und 26.2 ab. Bei dem Proximal-Gelenk 24 handelt es sich daher um ein Scheren-Drehgelenk.

Der rechte proximale Schenkel 16.2 besitzt einen ersten proximalen Teilschenkel 28.2 und einen zweiten proximalen Teilschenkel 30.2, die über ein proximales Ausgleichsgelenk 32.2 miteinander verbunden sind und die um eine proximale Ausgleichs-Gelenk-Drehachse A.2 gegeneinander verschwenkbar sind.

Auf gleiche Weise weist der rechte distale Schenkel 18.2 einen ersten distalen Teilschenkel 34.2. und einen zweiten distalen Teilschenkel 36.2 auf, die durch ein distales Ausgleichsgelenk 38.2 miteinander verbunden sind und gegeneinander um eine distale Ausgleichs-Gelenk-Drehachse U.2 schwenkbar sind. Die distale Ausgleichs-Gelenk-Drehachse U.2 verläuft ebenfalls parallel zu der Proximal-Gelenk-Drehachse P.

Die distalen Ausgleichsgelenke 38.1, 38.2 und die proximalen Ausgleichsgelenke 32.1 und 32.2. werden durch Scharniere gebildet. In einer alternativen Ausführungsform weisen die jeweiligen Schenkel 16.1, 16.2 bzw. 18.1, 18.2 im Bereich der Ausgieichsgelenke 32.1, 32.2 bzw. 38.1, 38.2 eine Materialverjüngung auf. Gemäß einer weiteren Alternative wird in dem entsprechenden Bereich ein Material mit einem geringeren Elastizitätsmodul verwendet.

Die distalen Schenkel 18.1 und 18.2 sind in einem Distal-Gelenk 40 um eine Distal-Gelenk-Drehachse Q schwenkbar miteinander verbunden und setzen sich in jeweilige Viertelschalen 42.1, 42.2 fort. Die Viertelschalen 42.1, 42.2 bilden eine Halbschale, mit der die Knieorthese 10 an einem nicht eingezeichneten Unterschenkel eines Patienten beispielsweise mit Bandagen befestigbar ist. Das Distal-Gelenk 40 ist wie das Proximal-Gelenk 24 aufgebaut und stellt ebenfalls ein Scheren-Drehgelenk dar.

Die Viertelschalen 26.1, 26.2 und 42.1, 42.2 sind aus einem flexiblen Material gebildet, so dass sie an die Gestalt des jeweiligen Körperglieds, an dem sie befestigt werden sollen, anpassbar sind. Die Schenkel 16.1, 16.2, 18.1, 18.2 sind beispielsweise als Kunststoff-Spritzgussteile gefertigt.

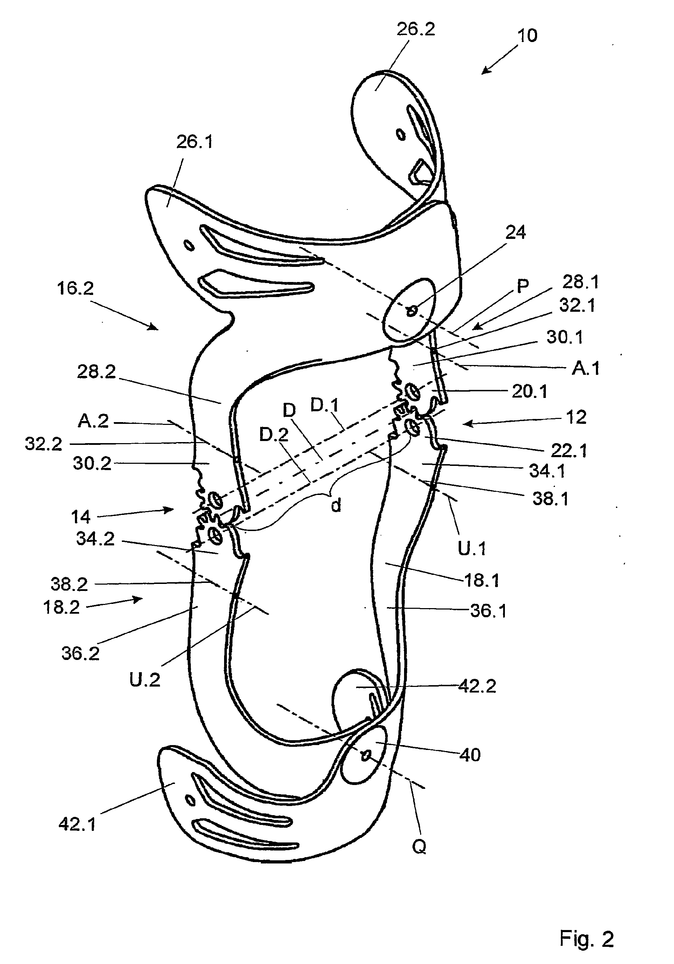

Figur 2 zeigt eine alternative Ausführungsform einer erfindungsgemäßen Knieorthese 10, bei der gleiche Bezugszeichen gleiche Komponenten bezeichnen. Im Unterschied zu der Knieorthese 10 gemäß Figur 1 ist das Proximal-Gelenk 24 als Drehgelenk, nicht aber als Scheren-Drehgelenk ausgebildet. Wenn sich daher der Abstand d zwischen dem linken Lastgelenk 12 und dem rechten Lastgelenk 14 verringert, so vergrößert sich der Abstand der beiden Viertelschalen 26.1 und 26.2. Gemäß weiterer erfindungsgemäßer Ausführungsformen kann auch das Distal-Gelenk 40 wie das Proximal-Gelenk 24 gemäß Figur 2 ausgebildet sein.

Zum Anpassen der Knieorthese 10 werden das Proximal-Gelenk 24 und das Distal-Gelenk 40, die beide als festlegbare Drehgelenke ausgebildet sind, gelöst und der Winkel zwischen den proximalen Schenkeln 16.1, 16.2 und den distalen Schenkeln 18.1, 18.2 wird so eingestellt, dass der Abstand d zwischen den Lastgelenken 12 und 14 die gewünschte Größe hat. Anschließend werden das Proximalgelenk 24 und das Distal-Gelenk 40 festgelegt und die Knieorthese 10 am Ober- bzw. Unterschenkel des Patienten befestigt.

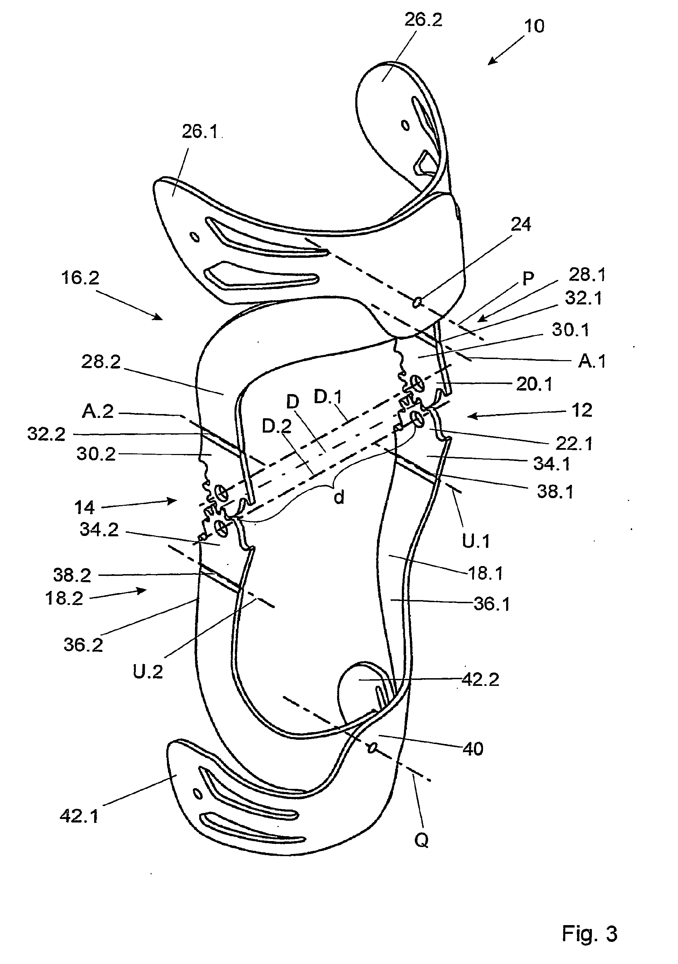

Figur 3 zeigt eine alternative Ausführungsform einer erfindungsgemäßen Knieorthese 10, bei der sowohl das Proximal-Gelenk 24 als auch das Distal-Gelenk 40 als Scheren-Drehgelenke ausgebildet sind. Die proximalen Ausgleichsgelenke 32.1, 32.2 und die distalen Ausgleichsgelenke 38.1, 38.2 sind durch Materialverjüngungen gebildet und können daher auch als Festkörpergelenke bezeichnet werden.

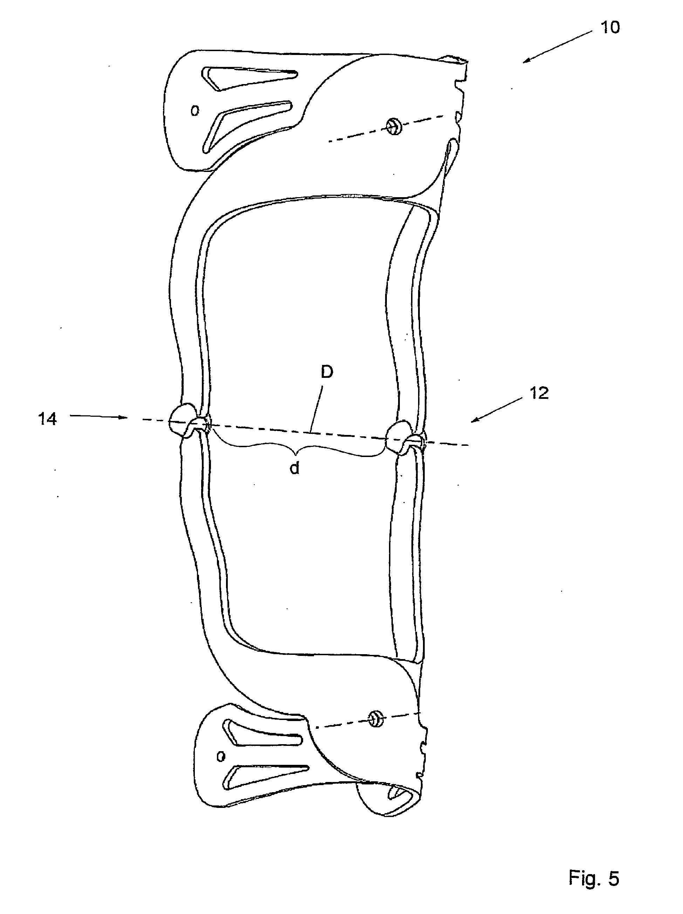

Figur 5 zeigt eine alternative Ausführungsform einer erfindungsgemäßen Knieorthese 10, bei der die Lastgelenke 12, 14 als Kugelgelenke ausgebildet sind und bei der daher Ausgleichsgelenke entbehrlich sind. An den Kugelgelenken sind nicht eingezeichnete Pelotten befestigt, um die Knieorthese 10 komfortabel am Körper des Patienten zu befestigen.

### Bezugszeichenliste

- 10: Knieorthese
- 12: linkes Lastgelenk
- 14: rechtes Lastgelenk
- 16.1, 16.2: proximaler Schenkel
- 18.1, 18.2: distaler Schenkel

- 20.1, 20.2: proximaler Gelenkkopf
- 22.1, 22.2: distaler Gelenkkopf
- 24: Proximal-Gelenk
- 26.1, 26.2: Viertelschale
- 28.1, 28.2: erster proximaler Teilschenkel

- 30.1, 30.2: zweiter proximaler Teilschenkel
- 32.1, 32.2: proximales Ausgleichsgelenk
- 34.1, 34.2: erster distaler Teilschenkel
- 36.1, 36.2: zweiter distaler Teilschenkel
- 38.1, 38.2: distales Ausgleichsgelenk

- 40: Distal-Gelenk
- 42.1, 42.2, 42.3, 42.4: Viertelschalen

- D: Lastgelenk-Drehachse
- D.1, D.2: Verbindungsgeraden
- P: Proximal-Gelenk-Drehachse
- A.2: proximale Ausgleichsgelenk-Drehachse
- U.2: distale Ausgleichsgelenk-Drehachse

- d: Abstand
- Q: Distal-Gelenk-Drehachse

## Patentansprüche

1. Orthese, insbesondere Knieorthese, mit zwei Lastgelenken (12, 14), die jeweils einen proximalen Schenkel (16.1, 16.2) und einen distalen Schenkel (18.1, 18.2) aufweisen,
**dadurch gekennzeichnet, dass**
(a) die proximalen Schenkel (16.1, 16.2) miteinander mittels eines Proximal-Gelenks (24) und die distalen Schenkel (18.1, 18.2) mittels eines Distal-Gelenks verbunden sind,
(b) wobei die proximalen Schenkel (16.1, 16.2) in dem Proximal-Gelenk (24) in Form eines Drehgelenks um eine Proximal-Gelenk-Schwenkachse (P) schwenkbar und gegeneinander festlegbar verbunden sind.

2. Orthese nach Anspruch 1, **dadurch gekennzeichnet, dass** die Proximal-Gelenk-Schwenkachse (P) durch das Proximal-Gelenk (24) verläuft.

3. Orthese nach Anspruch 2, **dadurch gekennzeichnet, dass** das Proximal-Gelenk (24) und/oder das Distal-Gelenk (40) ein Scheren-Drehgelenk, insbesondere ein festlegbares Scheren-Drehgelenk ist.

4. Orthese nach einem der Ansprüche 2 oder 3, **dadurch gekennzeichnet, dass** das Proximal-Gelenk (24) und/oder das Distal-Gelenk (40) um eine Drehgelenk-Schwenkachse (P, Q) schwenkbar ist, die senkrecht zu einer Lastgelenk-Schwenkachse verläuft, um die die beiden Lastgelenke (12, 14) schwenkbar sind.

5. Orthese nach Anspruch 4, **dadurch gekennzeichnet, dass** beide distalen Schenkel (18.1, 18.2)
(a) jeweils einen ersten distalen Teilschenkel (34.1, 34.2) und einen zweiten distalen Teilschenkel (36.1, 36.2) aufweisen und
(b) jeweils ein distales Ausgleichsgelenk (38.1, 38.2) umfassen, wobei die distalen Ausgleichsgelenke (38.1, 38.2) die jeweiligen Teilschenkel (34.1, 34.2, 36.1, 36.2) um eine Schwenkachse (U.1, U.2) schwenkbar miteinander verbinden, die parallel zu der Drehgelenk-Schwenkachse (Q) verläuft.

6. Orthese nach Anspruch 5, **dadurch gekennzeichnet, dass** beide proximalen Schenkel (16.1, 16.2)
(a) jeweils einen ersten Teilschenkel (28.1, 28.2) und einen zweiten Teilschenkel (30.1, 30.2) aufweisen und
(b) jeweils ein proximales Ausgleichsgelenk (32.1, 32.2) umfassen, wobei die proximalen Ausgleichsgelenke (32.1, 32.2) die jeweiligen Teilschenkel (28.1, 28.2, 30.1, 30.2) um eine Schwenkachse (A.1, A.2) schwenkbar miteinander verbinden, die parallel zu der Drehgelenk-Schwenkachse (P) verläuft.

7. Orthese nach einem der Ansprüche 5 oder 6, **dadurch gekennzeichnet, dass** die Ausgleichsgelenke (32.1, 32.2, 38.1, 38.2) Scharniergelenke sind.

8. Orthese nach einem der Ansprüche 5 oder 6, **dadurch gekennzeichnet, dass** die Ausgleichsgelenke (32.1, 32.2, 38.1, 38.2) durch Materialverjüngungen der jeweiligen Schenkel (16.1, 16.2, 18.1, 18.2) und/oder durch Materialien mit unterschiedlichen Festigkeitseigenschaften gebildet sind.

9. Orthese nach einem der vorstehenden Ansprüche, **dadurch gekennzeichnet, dass** die proximalen Schenkel (16.1, 16.2) vom jeweiligen Lastgelenk (12, 14) aus gesehen hinter dem Proximal-Gelenk (24) in eine Viertelschale (26.1, 26.2) auslaufen, so dass beide Viertelschalen eine proximale Halbschale zur Befestigung der Orthese (10) an einem Körperglied bilden.

10. Orthese nach einem der vorstehenden Ansprüche, **dadurch gekennzeichnet, dass** die distalen Schenkel (18.1., 18.2) vom jeweiligen Lastgelenk (12, 14) aus gesehen hinter dem Distal-Gelenk (40) in eine Viertelschale (42.1, 42.2) auslaufen, so dass beide Viertelschalen (42.1, 42.2) eine distale Halbschale zur Befestigung der Orthese (10) an einem Körperglied bilden.

11. Orthese nach Anspruch 9, **dadurch gekennzeichnet, dass** die proximalen Viertelschalen (26.1, 26.2) und/oder die distalen Viertelschalen (42.1, 42.2) flexibel sind, um eine Anpassung an das Körperglied zu ermöglichen.

12. Orthese nach einem der vorstehenden Ansprüche, **dadurch gekennzeichnet, dass** sie eine Rahmen-Knieorthese ist.

13. Orthese nach einem der vorstehenden Ansprüche, **dadurch gekennzeichnet, dass** die Lastgelenke monozentrisch sind.

14. Orthese nach einem der Ansprüche 1 bis 12, **dadurch gekennzeichnet, dass** die Lastgelenke polyzentrisch sind.

15. Orthese nach einem der Ansprüche 1 bis 11, 13 oder 14, **dadurch gekennzeichnet, dass** sie eine Ellenbogenorthese ist.

## Claims

1. Orthotic, in particular knee orthotic, with two joints (12, 14) each comprising a proximal leg (16.1, 16.2) and a distal leg (18.1, 18.2),
**characterised by**
a) the proximal leg (16.1, 16.2) being linked by a proximal joint (24) and the distal legs (18.1, 18.2) being linked by a distal joint,
b) wherein the proximal legs (16.1, 16.2) are linked in the proximal joint (24) which builds a pivoting joint for a fixable pivoting movement around a pivoting axis (P) of the proximal joint.

2. Orthotic of claim 1, wherein the pivoting axis (P) of the proximal joint runs through the proximal joint (24).

3. Orthotic of claim 2, wherein the proximal joint (24) and/or the distal joint (40) is/are a shear joint type pivoting joint or scissor type pivoting joint, in particular a fixable shear type pivoting joint or scissor type pivoting joint.

4. Orthotic of claim 2 or 3, wherein the proximal joint (24) and/or the distal joint (40) is/are pivotable around a pivoting axis (P, Q) of the pivoting joint having an orientation perpendicular to a pivoting axis of the joint for a pivoting movement of the two joints (12, 14).

5. Orthotic of claim 4, wherein the two distal legs (18.1, 18.2)
a) each comprise a first distal leg part (34.1, 34.2) and a second distal leg part (36.1, 36.2) and
b) each comprise a distal adjusting or balancing joint (38.1, 38.2) wherein the adjusting or balancing joints (38.1, 38.2)pivotably link respective leg parts (34.1, 34.2, 36.1, 36.2) for a pivoting movement around a pivoting axis (U.1, U.2), said pivoting axis having an orientation parallel to the pivoting axis (Q) of the pivoting joint.

6. Orthotic of claim 5, wherein the two proximal legs (16.1, 16.2)
a) each comprise a first leg part (28.1, 28.2) and a second leg part (30.1, 30.2) and
b) each comprise a proximal adjusting or balancing joint (32.1, 32.2) connecting the respective leg parts (28.1, 28.2, 30.1, 30.2) for a pivoting movement around a pivoting axis (A.1, A.2) having an orientation parallel to the pivoting axis (P) of the pivoting joint.

7. Orthotic of claim 5 or 6, wherein the adjusting or pivoting joints (32.1, 32.2, 38.1, 38.2) are hinge-joints.

8. Orthotic of claim 5 or 6, wherein the balancing or adjusting joints (32.1, 32.2, 38.1, 38.2) are build by a reduction of the cross section of the material of the respective leg (16.1, 16.2, 18.1, 18.2) and/or by materials of differing strength properties.

9. Orthotic of one of claims 1 to 8, wherein the proximal leg (16.1, 16.2) ends in a quarter shell behind the proximal joint (24) when seen from the respective joint (12, 14) such that the two quarter shells build a proximal half shell for mounting the orthotic (10) with a body part.

10. Orthotic of one of claims 1 to 9, wherein the distal legs (18.1, 18.2) end in a quarter shell (42.1, 42.2) behind the distal joint when seen from the respective joint (12, 14) such that the two quarter shells (42.1, 42.2) build a distal half shell for mounting the orthotic (10) with a body part.

11. Orthotic of claim 9, wherein the proximal quarter shells (26.1, 26.2) and/or the distal quarter shells (42.1, 42.2) are flexible for providing an adaptability to the body part.

12. Orthotic of one of claims 1 to 11, wherein the orthotic builds a frame-knee-orthotic.

13. Orthotic of one of claims 1 to 12, wherein the joints (12, 14) are mono-centrically.

14. Orthotic of one of claims 1 to 12, wherein the joints are poly-centrically.

15. Orthotic of one of claims 1 to 11, 13 or 14, wherein the orthotic is an orthotic for an elbow.

## Revendications

1. Orthèse, en particulier orthèse de genou, comportant deux articulations de charge (12, 14), qui comportent chacune une branche proximale (16.1, 16.2) et une branche distale (18.1, 18.2),
**caractérisée en ce que**
(a) les branches proximales (16.1, 16.2) sont reliées l'une à l'autre au moyen d'une articulation proximale (24), et les branches distales (18.1, 18.2) sont reliées l'une à l'autre au moyen d'une articulation distale,
(b) les branches proximales (16.1, 16.2) étant reliées de manière à pouvoir pivoter dans l'articulation proximale (24) formant une articulation rotoïde autour d'un axe de pivotement (P) de l'articulation proximale et de manière à pouvoir être immobilisées l'une par rapport à l'autre.

2. Orthèse selon la revendication 1, **caractérisée en ce que** l'axe de pivotement (P) de l'articulation proximale passe à travers l'articulation proximale (24).

3. Orthèse selon la revendication 2, **caractérisée en ce que** l'articulation proximale (24) et/ou l'articulation distale (40) sont des articulations rotoïdes extensibles, en particulier des articulations rotoïdes extensibles aptes à être immobilisées.

4. Orthèse selon la revendication 2 ou 3, **caractérisée en ce que** l'articulation proximale (24) et/ ou l'articulation distale (40) sont aptes à pivoter autour d'un axe de pivotement (P, Q) des articulations rotoïdes, lequel est orienté perpendiculairement à un axe de pivotement des articulations de charge, autour duquel peuvent pivoter les deux articulations de charge (12, 14).

5. Orthèse selon la revendication 4, **caractérisée en ce que** les deux branches distales (18.1, 18.2)
(a) comportent chacune une première portion distale (34.1, 34.2) et une deuxième portion distale (36.1, 36.2) et
(b) comportent chacune une articulation d'équilibrage distale (38.1, 38.2), lesdites articulations d'équilibrage distales (38.1, 38.2) reliant entre elles les portions (34.1, 34.2, 36.1, 36.2) respectives de manière à pouvoir pivoter autour d'un axe de pivotement (U.1, U.2), qui est parallèle à l'axe de pivotement (Q) de l'articulation rotoïde.

6. Orthèse selon la revendication 5, **caractérisée en ce que** les deux branches proximales (16.1, 16.2)
(a) comportent chacune une première portion proximale (28.1, 28.2) et une deuxième portion proximale (30.1, 30.2) et
(b) comportent chacune une articulation d'équilibrage proximale (32.1, 32.2), lesdites articulations d'équilibrage proximales (32.1, 32.2) reliant entre elles les portions (28.1, 28.2, 30.1, 30.2) respectives de manière à pouvoir pivoter autour d'un axe de pivotement (A.1, A.2), qui est parallèle à l'axe de pivotement (P) de l'articulation rotoïde.

7. Orthèse selon la revendication 5 ou 6, **caractérisée en ce que** les articulations d'équilibrage (32.1, 32.2, 38.1, 38.2) sont des articulations à charnière.

8. Orthèse selon la revendication 5 ou 6, **caractérisée en ce que** les articulations d'équilibrage (32.1, 32.2, 38.1, 38.2) sont formées par des rétrécissements de matière de la branche (16.1, 16.2, 18.1, 18.2) respective et/ou par des matériaux avec des propriétés de résistance différentes.

9. Orthèse selon l'une quelconque des revendications précédentes, **caractérisée en ce que** les branches proximales (16.1, 16.2), vues à partir de l'articulation de charge (12, 14) respective, se terminent en aval de l'articulation proximale (24) en formant un quart de coquille (26.1, 26.2), de telle sorte que les deux quarts de coquille forment une demi-coquille proximale destinée à la fixation de l'orthèse (10) sur un membre du corps.

10. Orthèse selon l'une quelconque des revendications précédentes, **caractérisée en ce que** les branches distales (18.1, 18.2), vues à partir de l'articulation de charge (12, 14) respective, se terminent en aval de l'articulation distale (40) en formant un quart de coquille (42.1, 42.2), de telle sorte que les deux quarts de coquille forment une demi-coquille distale destinée à la fixation de l'orthèse (10) sur un membre du corps.

11. Orthèse selon la revendication 9, **caractérisée en ce que** les quarts de coquille proximales (26.1, 26.2) et/ou les quarts de coquille distales (42.1, 42.2) sont flexibles, afin de permettre un ajustement au membre du corps.

12. Orthèse selon l'une quelconque des revendications précédentes, **caractérisée en ce qu'**il s'agit d'une orthèse de genou à cadre.

13. Orthèse selon l'une quelconque des revendications précédentes, **caractérisée en ce que** les articulations de charge sont monocentrées.

14. Orthèse selon l'une quelconque des revendications 1 à 12, **caractérisée en ce que** les articulations de charge sont polycentrées.

15. Orthèse selon l'une quelconque des revendications 1 à 11, 13 ou 14, **caractérisée en ce qu'**il s'agit d'une orthèse de coude.
